(19) Europäisches Patentamt
European Patent Office
Office européen des brevets

(11) Publication number: **0 392 697 A2**

(12) **EUROPEAN PATENT APPLICATION**

(21) Application number: **90303270.4**

(51) Int. Cl.[5]: **C12P 1/00, B01D 17/00**

(22) Date of filing: **28.03.90**

(30) Priority: **12.04.89 US 336985**

(43) Date of publication of application: **17.10.90 Bulletin 90/42**

(84) Designated Contracting States: **BE CH DE FR GB LI NL**

(71) Applicant: **PETROLITE CORPORATION**
**369 Marshall Avenue**
**Saint Louis Missouri 63119(US)**

(72) Inventor: **Keating, Richard D.**
**12004 Jacobson Court**
**Bridgeton, Missouri 63044(US)**

(74) Representative: **Seaborn, George Stephen et al**
**c/o Edward Evans & Co. Chancery House**
**53-64 Chancery Lane**
**London WC2A 1SD(GB)**

(54) **Demulsifiers for resolving emulsions formed in the production of pharmaceuticals.**

(57) A method for resolving emulsions produced in preparation of pharmaceuticals by fermentation. The method comprises adding an effective amount of a demulsifier to an emulsion that contains fermentation product that includes a pharmaceutical that is desired to be extracted from the emulsion. The demulsifier comprises an amine composition selected from the group consisting of (a) polyalkanolamine compositions comprising polyalkanolamines and having an average molecular weight of from about 325 to about 10,000 and a viscosity of from about 65 to about 7000 SUS (about 0.117 to about 15.4 stokes) when measured at 50% aqueous solution at 100° F (37.8° C), each of said polyalkanolemines having at least one amino group, (b) guaternary ammonium compositions formed by treating the polyalkanolamine compositions so as to quaternize at least some of the amino groups of the polyalkanolamines in the polyalkanolamine compositions, (c) salt compositions formed by treating the polyalkanolamine compositions so as to form the conjugate acid salts of at least some of the polyalkanolamines in the polyalkanolamine compositions, and (d) dilute forms of any of the polyalkanolamine compositions, the quaternary ammonium compositions and the salt compositions. Related compositions and methods are also disclosed.

EP 0 392 697 A2

# DEMULSIFIERS FOR RESOLVING EMULSIONS FORMED IN THE PRODUCTION OF PHARMACEUTICALS

## Background of the Invention

### 1. Field of the Invention

The present invention relates to demulsification, and more particularly to resolution of emulsions formed during the preparation of pharmaceuticals.

### 2. Description of the Prior Art

In a standard method for preparation of pharmaceuticals such as antibiotics, for example, penicillin or substances obtained from fungi (streptomycin, erythromycin, efrotomycin, and other mycins), a culture of bacteria or enzymes ferments a nutriment medium, such as vegetable oil in a water base, to produce a desired pharmaceutical. However, the fermented mass has been found to comprise not only the desired pharmaceutical, but also other organic fermentation products such as a biomass of bacteria or enzymes, unconverted nutriment medium, water and surfactant. Thus, the desired pharmaceutical must be extracted from the undesirable components of the fermented mass.

The desired pharmaceutical is extracted first by adjusting the pH of the fermented mass. Depending on the particular culture and desired pharmaceutical, the pH is adjusted either up or down, as necessary, by addition of a base or an acid, respectively, to precipitate the desired pharmaceutical. Shortly before or after adjustment of the pH, an extracting solvent, typically an organic base such as amyl acetate, methyl ethyl ketone, methyl isobutyl ketone, amyl alcohol, butyl alcohol, benzyl alcohol or the like, is added to extract the desired pharmaceutical from the water phase to the organic phase; and the phases are separated.

Often the water and organic phases are commingled in the form of an emulsion, and so separation can be difficult. It is believed that the organic phase typically is emulsified or dispersed in the water phase, although it is possible that the water phase is dispersed or emulsified through the organic phase or that actually an emulsion is dispersed or emulsified through another phase.

Nevertheless, depending on the components, some emulsions eventually break by themselves, that is, such emulsions have only "temporary stability" and over time and with the aid of gravity or centrifugation the emulsion separates into distinct layers. However, more commonly, a demulsifier is added to encourage or initiate separation by gravitation or centrifugation. Ordinarily, sodium chloride or another inorganic salt or a demulsifier such as DEMULSO I or III (trade designations of Petrolite Corp. for TEA condensates/esters and paraformaldehyde phenol resin ethoxylates, respectively) is employed as a demulsifier.

In any event, upon seperation, a solvent phase and a water phase are formed. The solvent phase comprises the desired pharmaceutical, solvent and small amounts of water and other impurities, such as organics of structure similar to that of the desired pharmaceutical and other undesirable components dissolved in the solvent. The water phase comprises the remaining components, including the biomass of the bacteria or enzymes, and a small amount of the desired pharmaceutical dissolved in the water and contained in the biomass.

The solvent phase may be cleaned further after separation from the water phase by adding clean water. By adjusting the pH of the mixture, the desired pharmaceutical sometimes can be shifted to either the water or the organic phase in order to simplify isolation of the pharmaceutical. By a second separation step, the desired phase may be isolated. If the addition of water forms a secondary emulsion, separation may be accomplished as described for the first emulsion.

When the phase containing the desired pharmaceutical has been cleaned to the desired level, excess solvent or water, depending on the phase in which the pharmaceutical is held, can be evaporated off.

The conventional method for demulsification and separation of phases has several drawbacks. For example, although inorganic salts are routinely used as demulsifiers, they do not perform as well as desired. Thus, the phase separation can take place more slowly than desired, and the separation tends to involve a relatively large, indistinct interface, with poor segregation of components into the separate phases. For example, a significant portion of the desired pharmaceutical is often entrapped within cells in the biomass. As a result, recovery of the pharmaceutical typically has been found to be as low as 80%.

Other demulsifiers have been used to resolve such emulsions, but are limited in that they resolve only certain of such emulsions. Moreover, although certain such demulsifiers resolve the phases more guickly,

clearly and thoroughly than do the inorganic salts, even better and faster phase separation is desirable. Further, demulsifiers that resolve such emulsions over the wide pH range exhibited by such emulsions are desired.

On the other hand various demulsifiers such as those discussed in US 4,505,839 to Bellos et al., and US 2,407,895 to Monson have been employed in resolving oil-in-water oil field emulsions. However, such oil field technology has not heretofore been applied in the radically different field of pharmaceutical preparation.

## Summary of the Invention:

Briefly, therefore, the present invention is directed to a novel method for resolving emulsions produced in preparation of pharmaceuticals by fermentation. The method comprises adding an effective amount of a demulsifier to an emulsion that contains fermentation product that includes a pharmaceutical that is desired to be extracted from the emulsion. The demulsifier comprises an amine composition selected from the group consisting of (a) polyalkanolamine compositions comprising polyalkanolamines and having an average molecular weight of from about 325 to about 10,000 and a viscosity of from about 65 to about 7000 SUS (about 0.117 to about 15.4 stokes) when measured at 50% aqueous solution at 100° F, (37.8° C), each of said polyalkanolamines having at least one amino group, (b) quaternary ammonium compositions formed by treating the polyalkanolamine compositions so as to quaternize at least some of the amino groups of the polyalkanolamines in the polyalkanolamine compositions, (c) salt compositions formed by treating the polyalkanolamine compositions so as to form the conjugate acid salts of at least some of the polyalkanolamines in the polyalkanolamine compositions, and (d) dilute forms of any of the polyalkanolamine compositions, the quaternary ammonium compositions and the salt compositions.

Among the several advantages found to be achieved by the present invention, therefore, may be noted the provision of a method for resolving emulsions produced during the processing of fermentation products which resolves the emulsion more quickly than do the demulsifiers conventionally employed; the provision of such method which resolves the emulsion more thoroughly than do the demulsifiers conventionally employed; the provision of such method which produces a more defined interface than do the demulsifiers conventionally employed; and the provision of such method which demulsifies a variety of such emulsions.

## Description of the Preferred Embodiments:

In accordance with the present invention, it has been discovered that demulsifiers comprising certain polyalkanolamine compositions having a viscosity in the range of from about 65 to about 7000 SUS (about 0.117 to about 15.4 stokes), when measured at 50% aqueous solution at 100° F (37.8° C), or their guaternary ammonium or conjugate acid salts, are surprisingly effective in resolving emulions produced in preparation of certain pharmaceuticals such as antibiotics, for example, penicillin or substances obtained from fungus (streptomycin, erythromycin, efrotomycin, etc.), by fermentation. Such demulsifiers have been found to be remarkably fast acting, with a nearly immediate phase separation, and to produce an exceptionally clear, well defined interface, with almost all of the desired pharmaceutical segregated into one of the phases. Not only that, but the demulsifiers of this invention have been found to be effective over a wide pH range and for a wide variety of emulsions produced in preparation of various pharmaceuticals by fermentation.

Suitable demulsifiers comprise or are derived from a polyalkanolamine composition that has an average molecular weight of from about 325 to about 10,000 and a viscosity of from about 65 to about 7000 SUS (about 0.117 to about 15.4 stokes) when measured at 50% aqueous solution at 100° F (37.8° C). Average molecular weight as used herein may be measured by osmometry, particularly vapor pressure osmometry, especially utilizing a molecular weight apparatus having suspended thermistors. Ak molecular weight apparatus such as Wescan Instruments, Inc. Model 233 may be employed. With respect to viscosity, by one method, to convert from SUS (Saybolt Universal Seconds) to stokes, for t SUS, where t is less than 100, stokes = 0.00226t - 1.95/t, and where t is greater than 100, stokes - 0.0022t - 1.35t. See also ASTM Designation D2162-82. Other discussions of osmometry may be found in Burge, D., "Molecular Weight Measurements by Osmometry," American Laboratory, June 1977; Morris, C.E.M., "Aspects of Vapor Pressure Osmometry", J. of Applied Polymer Science, Vol. 21, 435-448 (1977); and Van Dam, J., "Determination of Molecular Weights by Means of Thermoelectric Vapor Phase Osmometry", 83 Recueil, 129-140 (1964). Such polyalkanolamine compositions may be produced by the polymerization of alkanolamines.

The polymerization reaction may be aided by an iron or Lewis acid catalyst or, if desired, may be conducted at high temperatures, for example, about 275° C to about 300° C, without a catalyst. Suitable catalysts include iron and Lewis acids such as zinc halide, zinc halide/carboxylic acid, aluminum sulfate or aluminum sulfate/carboxylic acid. Preferably, water is removed throughout the polymerization reaction in order to produce a dehydration condensation product.

The reaction is halted before completion so that the reaction product, i.e., the polyalkanolamine composition, comprises not only polyalkanolamine, but also unpolymerized alkanolamine. Surprisingly, it has been found that this combination of components performs a variety of functions and so demulsifies in a manner far superior to the demulsification achieved by the individual components separately or by demlusifiers conventionally used in resolving emulsions produced in preparation of certain pharmaceuticals.

More specifically, upon application of the polyalkanolamine composition of this invention to such emulsions, the emulsion typically separates into distinct phases with a sharp, well defined interface almost immediately, and particulate matter in the emulsion being treated is sequestered into a tacky ball or layer that resides either at the interface or in the aqueous phase. The phase separation is almost complete, with nearly 100% of the separable water from the emulsion being treated residing in the aqueous phase, nearly 100% of the particulate matter sequestered in the tacky ball or layer and nearly 100% of the desired pharmaceutical residing in the organic phase. Thus, in view of the slight solubility of water in the organic and of the organic in water, generally at least about 98% of the total water from the emulsion is found in the aqueous phase.

It further has been found that not only can the condensed polyalkanolamine composition as produced by the polymerization reaction be utilized as a superior demulsifier, but so can the conjugate acid salts and quaternary ammonium salts thereof. The conjugate acid salts are formed by the addition of an acid to the polyalkanolamine composition. The quaternaries of such polyalkanolamines are produced by the further reaction of the polyalkanolamines with an alkyl halide having 1 to about 18 carbon atoms per molecule.

Relatively low molecular weight, i.e., 325 to 525, low viscosity, i.e., about 65 to about 150 SUS (about 0.117 to about 0.32 stokes) when measured at 50% aqueous solution at 100° F (37.8° C), polyalkanolamine compositions of this invention may be prepared in the general manner described in US 4,505,839 to Bellos et al. US 2,407,895 to Monson discusses the preparation of similar compositions. By carrying out the synthesis of US 4,505,839 at higher temperatures and for longer periods of time, higher molecular weight, i.e., up to 10,000, higher viscosity, i.e., about 700 to about 7000 SUS (about 1.54 to about 15.4 stokes) (when measured at 50% aqueous solution at 100° F (37.8° C)), polyalkanolamine compositions are formed. Surprisingly, whereas such relatively high viscosity compositions were avoided in preparation of the oil field demulsifiers of the US 2,407,895 and US 4,505,839 patents, such higher viscosity polyalkanolamine compositions have been found to be often even more effective than those described in the noted patents. Moreover, although the quaternary ammonium salts of the polyalkanolamines of this invention are particularly effective, it has also been discovered that the polyalkanolamines need not be quaternized to be effective.

In more detail, an alkanolamine is charged to a reactor vessel as and heated, preferably, in the presence of a catalyst. Desirably, the alkanolamine is a dialkanolamine such as diethanolamine or dipropanolamine or a trialkanolamine such as triethanolamine or tripropanolamine, but other alkanolamines, including but not limited to N,N-bis(2-hydroxyethyl)-N-hydroxypropylamine and even

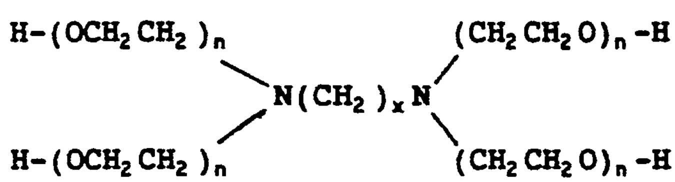

where n is an integer from 1 to about 10 and x is an integer from 1 to about 12, are also acceptable. Although diamines, triamines, and other such polyamines are appropriate, monoamines are preferred. Mixtures of alkanolamines are also acceptable. When the alkanolamine is a monoamine, it may be generally represented by the formula :

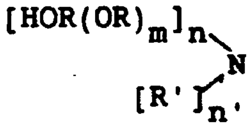

wherein OR is an alkylene oxide radical of at most about 4 carbon atoms, for example, a propylene oxide radical, butylene oxide radical, glycid radical or methyl glycid radical, but preferably is an ethylene oxide radical, $R'$ is hydrogen or an alkyl radical having at most about 6 carbon atoms, m is an integer of from 0 to 3, n is 2 or 3, and $n'$ is 0 or 1, provided however that $n+n'=3$. More preferably, the alkanolamine is diethanolamine or triethanolamine, most preferably, a trialkanolamine, especially triethanolamine. Also particularly preferred is N,N-bis(2-hydroxyethyl)-N-hydroxypropylamine.

The catalyst is of a type that aids in initiation of the polymerization of the alkanolamine. It is believed that iron or any Lewis acid is appropriate, but preferred catalysts are iron, zinc halide, zinc halide/carboxylic acid, aluminum sulfate and aluminum sulfate/carboxylic acid. Generally, the carboxylic acid is a lower carboxylic acid (i.e., at most about four carbon atoms), especially acetic acid, and the halide is chloride. Such catalysts are employed in a proportion of from about 1% to about 10% by weight, preferably from about 1% to about 3% by weight. The polymerization reaction is carried out at a temperature of about 180°C to about 300°C, preferably about 180°C to about 265° C. Water formed during the process and various decomposition products (e.g., acetaldehyde, ethylene glycol and ethanol) are removed continuously by condensation. Thus, the polymerization product is a dehydration condensation product.

Alternatively, the polymerization reaction may be conducted without a catalyst, or with a minimal amount (e.g., less than about 0.1% by weight) of catalyst. In such case, higher temperatures, that is, about 275°C to about 300°C, are employed.

The desired endpoint of the reaction may be determined either by measuring the amount of total water produced and removed or by monitoring the viscosity. Upon reaching the desired endpoint, the reaction is halted by removing the heat.

The resulting dehydration condensation reaction product has an average molecular weight of from about 325 to about 10,000 and a viscosity of from about 65 to about 7000 SUS (about 0.117 to about 15.4 stokes) when measured at 50% aqueous solution at 100°F (37.8 °C). The average molecular weight may be measured by osmometry as discussed above.

Although product at the lower end of the viscosity range is acceptable, a product resulting from a higher degree of cross-linking has been found to be more effective. Thus, a product having a viscosity of from about 700 to about 7000 SUS (about 1.54 to about 15.4 stokes) when measured at 50% aqueous solution at 100° F (37.8°C) is preferable. Products having an average molecular weight of from about 1000 to about 6000, especially from about 1000 to about 4000, and a viscosity of from about 700 to about 4000 SUS (about 1.54 to about 8.8 stokes), especially from about 700 to about 2000 SUS when measured at 50% aqueous solution at 100° F (37.8°C) are particularly desirable. This polymerization product has been found to be very water soluble.

It has also been found that when an iron catalyst is employed, the product tends to be of a highly piperazine structure, and that use of a zinc chloride catalyst yields a product comprises a moderate degree of piperazine structure mixed with morpholines. Use of an aluminum sulfate catalyst results in a piperazine and morpholine mixture of a higher morpholine to piperazine ratio than does use of the zinc chloride catalyst. On the other hand, employment of a zinc chloride/acetic acid catalyst produces a product having a piperazine content between that obtained from zinc chloride as the sole catalyst and that obtained from aluminum sulfate as the sole catalyst, and further comprising morpholines and esters of the acetic acid. The ester formation has been found to limit the degree of cross-linking.

As noted, the polymerization product may be used at full strength or in a diluted form as a demulsifier for resolution of emulsions formed during the preparation of pharmaceuticals. For example, the polymerization product can be diluted to one-half strength (by weight) with a solvent such as water, an alcohol, for example, methanol or propanol, or a mixture of water and alcohol for shipment and then, if desired, diluted further for application. If desired, the polymerization product may be regained from the dilute form by removing the solvent therefrom, for example, by evaporation or distillation. A product of approximately the same viscosity and average molecular weight of the polymerization product can thereby be obtained in this manner. Other additives incorporated into the product may also be present.

Alternatively, however, the polymerization product may be converted to its conjugate acid salt by addition of an acid, for example, acetic acid, hydrochloric acid, phosphoric acid or sulfuric acid. As with the untreated polymerization product, the salt may then be used directly as a demulsifier or diluted and then

applied.

Yet another alternative is to convert the polymerization product to a quaternary ammonium salt. The quaternary ammonium salt may be formed by first diluting the polymerization product with water or alcohol, or both, and adding an alkyl halide. The alkyl halide is slowly metered in to a continuously stirred batch of the polymerization product.

Generally, an alkyl group of the alkyl halide has from 1 to about 18 carbon atoms, although lower alkyl groups, i.e., those having from 1 to about 8 carbon atoms, are preferred, and methyl halides, ethyl halides and propyl halides are most preferred, especially methyl chloride, ethyl chloride and propyl chloride. The degree of quaternization can be selected by controlling the relative proportion of alkyl halide to polymerization product. For example, 50% quaternization may be obtained by mixing one-half equivalents of alkyl halide per equivalent of nitrogen atoms in the polymerization product. Alternatively, a polyalkanolamine composition of a specified degree of quaternization may be obtained by mixing polyalkanolamine compositions of different degrees of quaternization. For example, a polyalkanolamine composition of 50% quaternization may be prepared by mixing equal proportions of a polyalkanolamine composition of 75% quaternization and a polyalkanolamine composition of 25% quaternization. Generally, quaternized product is not quaternized 100%, and the unquaternized polymers of the product as well as the quaternized polymers of the product have been found to play an active role in demulsification.

Preferably the degree of quaternization is from about 25% and 100%, more preferably about 70% to about 80%, especially about 75%, based on number of nitrogen atoms quaternized. As with the unquaternized polymerization product and the acid salt thereof, the quaternary ammonium salt may then be used directly as a demulsifier for resolution of emulsions formed during the preparation of pharmaceuticals or diluted and then applied as a demulsifier for resolving such emulsions. If desired, the pH of many of the products may be altered to a degree to limit its affect on the pH of the emulsion being treated. The pH of highly quaternized compositions can be adjusted to a significant degree while retaining efficacy.

The emulsions to which the demulsifiers of this invention are applicable are those formed during preparation of pharmaceuticals such as antibiotics, for example, penicillin or substances obtained from fungi (streptomycin, erythromycin, efrotomycin, etc.), in which a culture of bacteria or enzymes ferments a nutriment medium, such as vegetable oil in a water base, to produce a desired pharmaceutical. The emulsion comprises an organic solvent, typically methyl isobutyl ketone, emulsifiers, nutrient from the nutriment medium, particulate matter including enzymes or bacteria, and various electrolytes. Such emulsions are produced in a broad range of pH's and the components are present in a variety of relative proportions.

To resolve such emulsions, the demulsifier is applied to the emulsion by addition of an amount corresponding to from about 100 parts by weight and about 10,000 parts by weight of the polyalkanolamine composition to one million parts by weight of the emulsion. If desired, the demulsifier may be mixed into the emulsion by shaking or stirring. Preferably from about 200 parts by weight to about 1000 parts by weight of the polyalkanolamine composition to one million parts by weight of the emulsion is added, but about 1000 parts by weight to about 4000 parts by weight or about 4000 parts by weight to about 10,000 parts by weight are also acceptable.

The optimal proportion depends on the particular emulsion being treated. Such emulsions typically comprise about 25% by weight to about 75% by weight, often about 50% by weight solvent, commonly methyl isobutyl ketone and water. Some such emulsions have been neutralized to a pH of from about 7 to about 7.4. However, other emulsions have other pH's, and it has been found that the demulsifiers of this invention are applicable over a wide pH range -- at least from about 3 to about 9. The pH of the demulsifier may be adjusted by addition of an acid or base, as desired, to approximate the pH of the emulsion and so avoid altering the pH of the emulsion. The cationic character of the demulsifiers of this invention is believed to play a significant role in demulsification. Quaternized product tends to retain its cationic nature at relatively high pH's, i.e., pH's above about 10. However, the acid salt demulsifiers tend to become nonionic at relatively high pH's while remaining effective. Thus, it is believed that such demulsifiers function in some manner other than by means of charge or charge density under such conditions, perhaps by some mechanism related to the complexity of the demulsifier structure.

The pH may be adjusted by addition of an acid or base, as necessary. Other additives, such as zinc chloride, salts or organics also may be incorporated into the demulsifier, if desired.

It has been found that upon addition of the demulsifier, the emulsion typically separates almost immediately into two phases with a sharp, well-defined interface, with an agglomeration of the solids in the mixture either at the interface or at the bottom of the mixture.

The following examples describe preferred embodiments of the invention. Other embodiments within the scope of the claims herein will be apparent to one skilled in the art from consideration of the

specification or practice of the invention as disclosed herein. It is intended that the specification, together with the examples, be considered exemplary only, with the scope and spirit of the invention being indicated by the claims which follow the examples. In the examples all percentages are given on a weight basis unless otherwise indicated.

## EXAMPLE 1

Rich extract was obtained from the production of an antibiotic derived from fungus. The unfiltered fermentation broth was acidified and contacted with methyl isobutyl ketone as an extracting solvent to produce the rich extract. The rich extract was diluted with deionized water and neutralized with sodium hydroxide to a pH of about 7 to about 7.4, and separated into several emulsion test samples of 40 to 50 ml each.

All tests were carried out in centrifuge tubes For each test, demulsifier was added as a 1% aqueous (vol/vol) solution and mixed into the emulsions with hand shaking or with the aid of a tube mixer. Some samples were sheared with a small homogenizer to simulate the high shear the emulsion will encounter as it enters the disk certrifuge. All samples were centrifuged for five minutes at 3200 rpm. The tubes were then judged on the amount (percent) of solvent and water recovered, the clarity of the two phases and the amount and relative location of solids in the tubes.

Test samples were run with a selection of compounds. The demulsifiers tested are designated as follows (with viscosity measured in SUS at 50% solution of the polymer composition in water at 100° F (37.8° C)):

| Demulsifier | Composition | Viscosity |
|---|---|---|
| A | poly(quaternary polytriethanolamine) | 700-4,000 |
| B | poly(quaternary polytriethanolamine) | 65-150 |
| C* | polymerization product of urea and amine | |
| D* | dithiocarbamate | |

* Demulsifier not within the scope of this invention.
Demulsifiers A and B were prepared in accordance with the procedures outlined in Bellos et al. US 4,505,839. The condensation reactions were allowed to proceed until the desired viscosities were achieved.

The compounds were tested at doses of 250 and 500 ppm, based on the total emulsion volume, and ranked in order of aqueous phase clarity and solvent phase clarity, with the numeral 1 indicating the clearest sample and 4 indicating the least clear sample. The emulsion pH of the samples was 7.1. The results were as follows:

| 250 ppm dose of demulsifier: | | | |
|---|---|---|---|
| Demulsifier | Aqueous Phase Clarity (Rank) | Solvent Phase Clarity (Rank) | Comments |
| A | 4 | 3 | thin interface |
| B | 2 | 2 | thin dark interface |
| C | 1 | 1 | bad cloudy interface |
| D | 3 | 4 | bad break; cloudy interface |

| 500 ppm dose of demulsifier: | | | |
|---|---|---|---|
| Demulsifier | Aqueous Phase Clarity (Rank) | Solvent Phase Clarity (Rank) | Comments |
| A | 2 | 3 | thin dark interface |
| B | 1 | 2 | drops solids to bottom |
| C | 3 | 1 | thin interface |
| D | 4 | 4 | bad break; cloudy interface |

Of particular significance to the quality of resolution is the nature of the interface, that is, that the interface be very thin, and the aggregation of solids. The compounds were compared with a blank and samples dosed with NaCl and D5430, a quaternary fatty acid amine salt from Akzo Chemicals, Inc. The emulsion pH of the samples was 7.1. The solvent phase clarity was ranked as described with the above samples. The results of these tests were as follows:

| | | Aqueous | | |
|---|---|---|---|---|
| Demulsifier | Dose (ppm) | Phase Clarity | Solvent Phase Clarity (Rank) | Comments |
| B | 300 | sl. cloudy | 2 | good interface |
| B | 500 | better | 3 | good interface |
| B | 700 | better | * | |
| A | 300 | excellent | 1 | excellent interface |
| A | 500 | excellent | 5 | excellent interface |
| A | 700 | excellent | * | excellent interface |
| D5430 | 500 | sl. cloudy/dark | 4 | good aq. phase, but a lot of solids in solvent |
| Nothing | | | good | bad interface into solvent; fair aq. phase |
| 1% NaCl | | cloudy | good | large interface |

*Showed signs of overtreatment (cloudiness).

Solids dropped to the bottom in these samples. Demulsifier A showed the best results at lower doses and perhaps indicated signs of overtreatment, for example, exhibiting cloudiness, at 700 ppm. This compound also appeared to be most effective at water-wetting the solids and dropping them to the bottom of the aqueous phase. The D5430 produced relatively clear water but appeared to leave traces of solids or water in the solvent layer. The sample treated with the brine left a large interface. Additional tests were conducted to confirm the results on a homogenized emulsion; as shown below, Demulsifier A produced clear solvent phases under the more rigorous conditions. A final test to determine the effect of strong caustic (10% NaOH) on the demulsifiers also showed favorable results.

| Demulsifier | Dose (ppm) | Aqueous Phase Clarity | Solvent Phase Clarity | Interface | Comments |
|---|---|---|---|---|---|
| A | 200 | bad | v. good | good | * |
| A | 400 | best | v. good | good | * |
| A | 600 | bad | v. good | good | * |
| A | 800 | bad | v. good | v. good | * |
| A | 1000 | bad | v. good | v. good | * |
| Nothing | | bad | v. good | v. poor 25ml. | * |
| Nothing | | bad | v. good | v. poor 25ml. | * |
| | | (Rank) | (Rank) | | |
| A | 400** | 1 | 3 | Best water | |
| A | 400*** | 1 | 4 | Best water | |

*Acceptable solids at interface.
**Not homogenized.
***Homogenized.

EXAMPLE

A second round of tests was conducted according to the method of Example 1. On an emulsion which contained a much higher level of solids than employed in the tests of Example 1. Demulsifier A was shown to perform well at higher doses, up to 1000 ppm (based on total emulsion) at pH 7.2 and 7.4. The results for Demulsifier A were as follows:

| Dose (ppm) | pH | Solvent Phase | | Comments |
|---|---|---|---|---|
| | | Recovery (ml) | Clarity | |
| 500 | 7.4 | 20 | good | About 7 ml. interface |
| 1000 | 7.4 | 25 | good | Good aq. phase clarity |
| 1500 | 7.4 | 25 | good | Slightly turbid aq. phase |

This test was repeated on an emulsion which was homogenized for 30 minutes to simulate an extreme case of shear, and the product still provided good results.

Additional tests run on a homogenized sample of rich extract (from Example 1) which contained a very high level of solids (perhaps 1-2% or more) indicated that at pH 7.1 good solvent quality could be obtained at 600 ppm but that the water quality suffered, presumably due to the solids.

The same emulsion at pH 7.4 showed a clean break with Demulsifier A at 800-1000 ppm when run at 70°F (21°C) and 120°F (49°C). The addition of heat appeared to have no significant effect on the resolution of the emulsion.

In view of the above, it will be seen that the several advantages of the invention are achieved and other advantageous results attained.

As various changes could be made in the above methods and compositions without departing from the scope of the invention, it is intended that all matter contained in the above description shall be interpreted as illustrative and not in a limiting sense.

**Claims**

1. A method for resolving emulsions produced in preparation of pharmaceuticals by fermentation comprising adding an effective amount of a demulsifier to an emulsion that contains fermentation product that includes a pharmaceutical that is desired to be extracted from the emulsion, said demulsifier comprising an amine composition selected from the group consisting of (a) polyalkanolamine compositions comprising polyalkanolamines and having an average molecular weight of from about 325 to about 10,000 and a viscosity of from about 65 to about 7000 SUS (about 0.117 to about 15.4 stokes) when measured at 50% aqueous solution at 100° F (37.8° C), each of said polyalkanomines having at least one amino group, (b) guaternary ammonium compositions formed by treating said polyalkanolamine compositions so as to quaternize at least some of the amino groups of the polyalkanolamines in said polyalkanolamine compositions, (c) salt compositions formed by treating said polyalkanolamine compositions so as to form the conjugate acid salts of at least some of the polyalkanolamines in said polyalkanolamine compositions, and (d) dilute forms of any of said polyalkanolamine compositions, said quaternary ammonium compositions and said salt compositions.

2. A method as set forth in claim 1 wherein said demulsifier was prepared by diluting said amine composition with a solvent selected from the group consisting of water, alcohols and mixtures thereof.

3. A method as set forth in claim 1 wherein said polyalkanolamine compositions were prepared by condensation polymerizing an alkanolamine.

4. A method as set forth in claim 3 wherein said polyalkanolamine compositions further comprise unpolymerized alkanolamine.

5. A method as set forth in claim 4 wherein said polymerization is a catalyzed reaction carried out by polymerizing the alkanolamine in the presence of a catalyst selected from the group consisting of iron and Lewis acids.

6. A method as set forth in claim 5 wherein said catalyst is selected from the group consisting of iron, zinc halide, zinc halide/carboxylic acid, aluminum sulfate and aluminum sulfate/carboxylic acid.

7. A method as set forth in claim 6 wherein said catalyst is selected from the group consisting of iron, zinc chloride, zinc chloride/acetic acid, aluminum sulfate and aluminum sul fate/acetic acid and wherein the alkanol groups of the alkanolamine reactant are alcohols having 1 to 4 carbon atoms.

8. A method as set forth in claim 7 wherein said alkanolamine is selected from the group consisting of diethanolamine, dipropanolamine, triethanolamine, tripropanolamine, N,N-bis(2-hydroxyethyl)- N-hydroxypropylamine, and mixtures thereof.

9. A method as set forth in claim 1 wherein said polyalkanolamine composition has a viscosity of from about 700 to about 7000 SUS (about 1.54 to about 15.4 stokes) when measured at 50% aqueous solution at 100° F (37.8° C).

10. A method as set forth in claim 9 wherein said polyalkanolamine composition has an average molecular weight of from about 1000 to about 10,000.

11. A method as set forth in claim 10 wherein said polyalkanolamine composition has a viscosity of from about 700 to about 4000 SUS (about 1.54 to about 8.8 stokes) when measured at 50% aqueous solution at 100° F (37.8° C).

12. A method as set forth in claim 11 wherein said polyalkanolamine composition has an average molecular weight of from about 1000 to about 6000.

13. A method as set forth in claim 12 wherein said guaternary ammonium compositions were produced by condensation polymerizing an alkanolamine to produce a polyalkanolamine precurser, and then reacting the precursor with an alkyl halide having 1 to about 18 carbon atoms.